(19) Europäisches Patentamt
European Patent Office
Office européen des brevets

(11) **EP 1 755 514 B1**

(12) **EUROPEAN PATENT SPECIFICATION**

(45) Date of publication and mention
of the grant of the patent:
**01.12.2010 Bulletin 2010/48**

(51) Int Cl.:
*A61F 13/20* (2006.01)     *A61F 13/15* (2006.01)
*D04H 1/22* (2006.01)

(21) Application number: **05749337.1**

(22) Date of filing: **13.05.2005**

(86) International application number:
**PCT/US2005/017113**

(87) International publication number:
**WO 2005/112860 (01.12.2005 Gazette 2005/48)**

(54) **INTRAVAGINAL DEVICE WITH FLUID TRANSPORT PLATES AND METHODS OF MAKING**

INTRAVAGINALE VORRICHTUNG MIT FLÜSSIGKEITSTRANSPORTPLATTEN UND
HERSTELLUNGSVERFAHREN

DISPOSITIF INTRAVAGINAL DOTE DE PLAQUES DE TRANSPORT DE FLUIDE ET PROCEDES
DE FABRICATION

(84) Designated Contracting States:
**AT BE BG CH CY CZ DE DK EE ES FI FR GB GR
HU IE IS IT LI LT LU MC NL PL PT RO SE SI SK TR**

(30) Priority: **14.05.2004 US 572055 P
14.05.2004 US 848257
14.05.2004 US 847951**

(43) Date of publication of application:
**28.02.2007 Bulletin 2007/09**

(73) Proprietor: **Johnson and Johnson Consumer
Companies, Inc.
Skillman, NJ 08558-9418 (US)**

(72) Inventors:
• **BINNER, Curt**
**SOMERSET, New Jersey 08873 (US)**
• **CARASSO, Samuel C**
**MILLTOWN, New Jersey 08850 (US)**
• **CHASE, David**
**Somerville**
**New Jersey 08876 (US)**
• **DANYI, Erin**
**Nicholasville**
**Kentucky 40356 (US)**
• **GLASGOW, Tara**
**New Hope**
**Pennsylvania 18938 (US)**
• **KIMBALL, David L**
**Flemington**
**New Jersey 08822 (US)**
• **KOZOROVITSKY, Julia**
**Longhorne**
**Pennsylvania 19047 (US)**
• **NG, Tony C**
**East Brunswick**
**New Jersey 08816 (US)**

(74) Representative: **Kirsch, Susan Edith et al
Carpmaels & Ransford
One Southampton Row
London
WC1B 5HA (GB)**

(56) References cited:
**WO-A-01/01906      WO-A-97/09017
WO-A-99/00096      US-B1- 6 177 608**

# Description

## Field of the Invention

The present invention relates to devices for capturing and storing body fluid intravaginally. More particularly, the present invention relates to device for capturing body fluid intravaginally via a fluid transport element and transporting the body fluid to a fluid storage element where the fluid is stored. Additionally, this application relates to methods of making such devices

## Background of the Invention

Devices for capturing and storing bodily fluid intravaginally are commercially available and known in the literature. Intravaginal tampons are the most common example of such devices. Commercially available tampons are generally compressed cylindrical masses of absorbent fibers that may be overwrapped with an absorbent or nonabsorbent cover layer.

The tampon is inserted into the human vagina and retained there for a time for the purpose of capturing and storing intravaginal bodily fluids, most commonly menstrual fluid. As intravaginal bodily fluid contacts the tampon, it should be absorbed and retained by the absorbent material of the tampon. After a time, the tampon and its retained fluid is removed and disposed, and if necessary, another tampon is inserted.

A drawback often encountered with commercially available tampons is the tendency toward premature failure, which may be defined as bodily fluid leakage from the vagina while the tampon is in place, and before the tampon is completely saturated with the bodily fluid. The patent art typically describes a problem believed to occur that an unexpanded, compressed tampon is unable to immediately absorb fluid. Therefore, it presumes that premature leakage may occur when bodily fluid contacts a portion of the compressed tampon, and the fluid is not readily absorbed. The bodily fluid may bypass the tampon.

To overcome this problem of premature leakage, extra elements have been incorporated into a basic tampon to try to direct and control the flow of fluid toward the absorbent core.

For example, US Pat. No. 4,212,301 (Johnson) discloses a unitary constructed digital tampon having a lower portion compressed preferably in the radial direction to form a rigid, rod-like element, which provides a central rigidified elongated core and an upper portion left substantially uncompressed. After insertion, the uncompressed portion may be manipulated to contact the vaginal wall to provide an immediate seal against side leakage. The uncompressed portion allows for high absorbent capacity immediately upon insertion. While this tampon may allow for a certain amount of protection from bypass leakage, the uncompressed portion may become saturated before the compressed portion has a chance to expand and become absorbent.

US Pat. No. 6,358,235 (Osborn et al.) discloses a "hollow" bag-like tampon that may have an interior projection made from highly compressed absorbent material. The interior projection is preferably attached to the inside surface of the head of the tampon. The hollow tampon portion may include at least one pleat in the absorbent outer surface and is soft and conformable. The tampon is not precompressed to the point where the fibers temporarily "set" and re-expand upon the absorption of fluid. The absorbent portions of the tampon can saturate locally, which leads to bypass leakage.

US Pat. No. 6,177,608 (Weinstrauch) discloses a tampon having nonwoven barrier strips that are outwardly spreadable from the tampon surface to reliably close the free spaces believed to exist within a vaginal cavity. The nonwoven barrier strips extend about the tampon in a circumferential direction at the surface or in a helical configuration about the tampon and purportedly conduct menstrual fluid toward the tampon surface. The nonwoven barrier strips are attached to the cover by means of gluing, heat bonding, needle punching, embossing or the like and form pleats. The nonwoven barrier strips are attached to the tampon blank and the blank is embossed, forming grooves extending in a longitudinal direction. While this tampon purports to direct fluid to the core, it attempts to achieve this by forming pockets of absorbent nonwoven fabric. In order to function, it appears that these pockets would have to be opened during use to allow fluid to enter. However, based upon current understandings of vaginal pressures, it is not understood how the described structure could form such an opened volume.

US Pat. No. 6,206,867 (Osborn) suggests that a desirable tampon has at least a portion of which is dry expanding to cover a significant portion of the vaginal interior immediately upon deployment. To address this desire, it discloses a tampon having a compressed central absorbent core having at least one flexible panel attached along a portion of the side surface of the core. The flexible panel appears to provide the "dry-expanding" function, and it extends outwardly from the core away from the point of attachment. The flexible panel contacts the inner surfaces of the vagina when the tampon is in place and purportedly directs fluid toward the absorbent core. The flexible panel is typically attached to the pledget prior to compression of the pledget to form the absorbent core and remains in an uncompressed state.

US Pat. No. 5,817,077 (Foley et al.) discloses a method of preserving natural moisture of vaginal epithelial tissue while a using a tampon where the tampon has an initial capillary suction pressure at the outer surface of less than about 40 mm Hg. This allows the tampon to absorb vaginal secretions without substantially drying the vaginal epithelial tissue. The multiple cover layers can be used to increase the thickness of the cover material. While this represents a significant advancement in the art, this invention does not address by-pass leakage.

Additionally, US Pat. No. 5,545,155 (Hseih et al.) discloses an external absorbent article that has a set of plates separated by spacer elements. The plates may be treated to affect wettability so that fluid will flow easily across the surface. Extending through the upper plate is a plurality of openings, which allow fluid to flow with little restriction into the space between the upper and lower plates. When the fluid flows downward in the z-direction from the upper plate to the lower plate, it will then flow laterally in the x- and y-directions. Therefore, this external absorbent article can contain fluid gushes, but it does not appear to address the problems relating in particular to intravaginal devices, such as a tampon.

While the prior art is replete with examples of sanitary protection articles that capture bodily fluids both externally and intravaginally, these examples do not overcome the problem of premature failure often identified as by-pass leakage that commonly occurs while using internal sanitary protection devices. Many solutions to this problem have involved increasing the rate of expansion of a highly compressed absorbent article.

## Summary of the Invention

We have found a novel way to address the problem of premature product failure. This invention is not dependent on the expansion of the compressed absorbent but rather incorporating an element, which is adaptable to the vagina. In our invention, we increase the contact area of the absorbent device and thereby reduce by-pass leakage.

A first aspect of the invention provides a fluid management device (10) for use in a mammalian body, the device comprising:

    a) a fluid storage element having a longitudinal axis, an insertion end and a withdrawal end; and
    b) at least one fluid transport element being in fluid communication with the fluid storage element, the at least one fluid transport element comprising:

       (i) a first plate having an outwardly oriented surface and an inwardly oriented surface;
       (ii) a second plate coupled to the first plate; that has a first surface disposed and maintained in facing relationship with the inwardly oriented surface of the first plate and an opposite surface; and that is capable of separating from the first plate sufficiently to provide inter-plate capillary action;

    wherein the at least one fluid transport element is bendable about an axis substantially parallel to the longitudinal axis of the fluid storage element.

The fluid transport element may substantially envelop the fluid storage element, and it may be attached to the withdrawal end of the fluid storage element, on at least one longitudinal side of the fluid storage element, to itself proximate the withdrawal end of the fluid storage element, and/or to the withdrawal string.

Another aspect of the invention relates to a method of producing an intravaginal device comprising the steps of:

    a) separating an individual sheet from a supply of material having properties useful to move bodily fluids;
    b) providing a fluid storage element having a longitudinal axis, longitudinal sides, an insertion end and a withdrawal end;
    c) engaging the individual sheet with the insertion end of the fluid storage element and with pleating edges of forming blades;
    d) urging the individual sheet through a forming tool with relative movement of the forming tool in relation to the fluid storage element and the pleating edges of forming blades;
    e) bonding at least a portion of the individual sheet to the fluid storage element to form at least a portion of the individual sheet into at least one fluid transport element capable of extending radially away from the fluid storage element;
    f) folding the at least one fluid transport element about an axis parallel to longitudinal axis (x-x) of the fluid storage element; and
    g) packaging the resultant intravaginal device.

A further aspect of the invention relates to an apparatus for producing an intravaginal device comprising:

    a) a forming tool comprising:

       (i) a holding plate having a plurality of vacuum ports formed in a face thereof;
       (ii) a substantially circular aperture disposed on the plate having a plurality of slots connected to and extending therefrom;

    b) a male tool comprising a plurality of forming blades, each blade having a guide edge, arranged radially about an aperture;
    c) at least one bonding element moveable toward the aperture in the forming tool; and
    d) at least one pushrod moveable through the apertures of each of the forming tool and the male tool; wherein the apertures of the forming tool and male tool are aligned along a machine axis to permit the pushrod to move fluid storage element through each apertures, the forming blades of the male tool are aligned with the slots of the forming tool, and the guide edges of the forming blades are arranged and configured to accommodate a fluid storage element aligned with the aperture of the male tool.

Other aspects and features of the present invention will become apparent to those ordinarily skilled in the art upon review of the following description of specific embodiments of the invention in conjunction with the accompanying drawings.

**Brief Description of the Drawing**

Fig 1a shows a side elevation of an intravaginal device having a fluid transport element in fluid communication with a fluid storage element.

Fig. 1b shows a cross-sectional view of the device in Fig. 1 a taken along line b-b.

Fig. 1c shows the transverse cross-section shown in 1b, after the introduction of a fluid between the plates of the fluid acquisition element.

Fig 2 shows an enlarged cross-section of an embodiment of a fluid transport element of the present invention having nubbles to separate a set of film plates.

Figs. 3a-c show enlarged cross-sections of alternate embodiments of fluid transport elements of the present invention formed of polymeric apertured formed film having differing orientations of the formed film plates.

Figs. 4a-e show various aspects and orientations of an intravaginal device of the present invention.

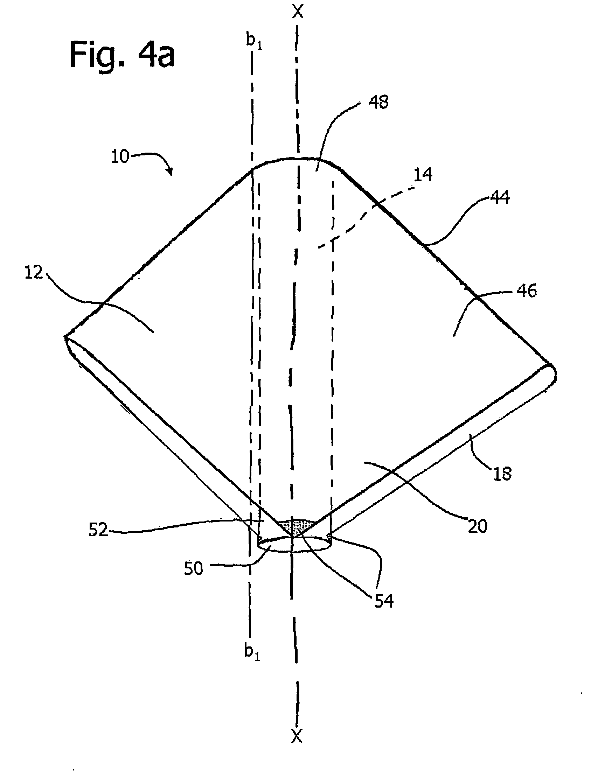

Fig. 4a shows a perspective view of a tampon having a plurality of fluid transport elements extending therefrom that are formed from a folded sheet material.

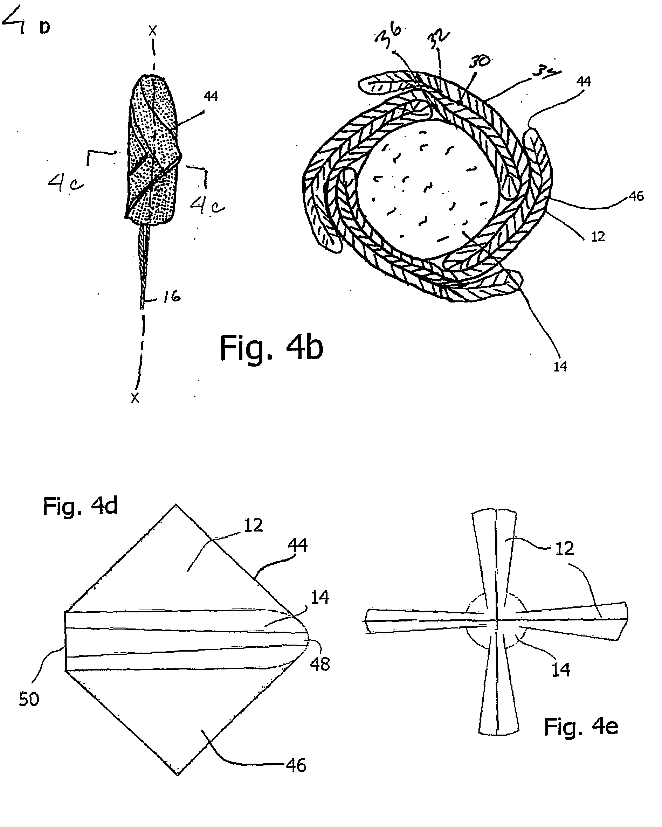

Fig. 4b shows a side elevation of the tampon with a plurality of fluid transport elements wrapped around the fluid storage element.

Fig. 4c shows a transverse cross-section along line 4c-4c in Fig. 4b. Fig. 4d shows a side elevation of the tampon of Fig. 4a.

Fig. 4e shows a top elevation of the tampon of Fig. 4a.

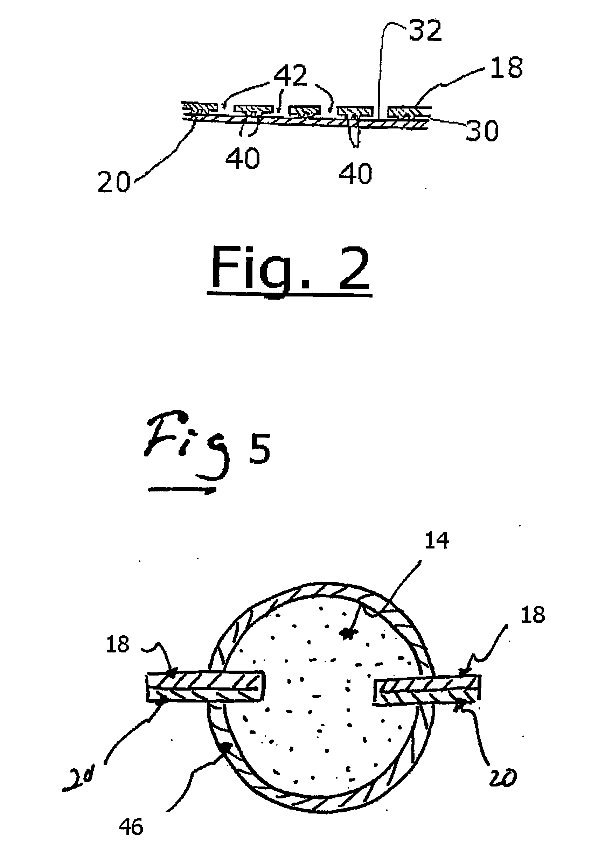

Fig. 5 shows a transverse cross-section of an alternate embodiment having a pair of fluid transport elements partially extending into the storage element.

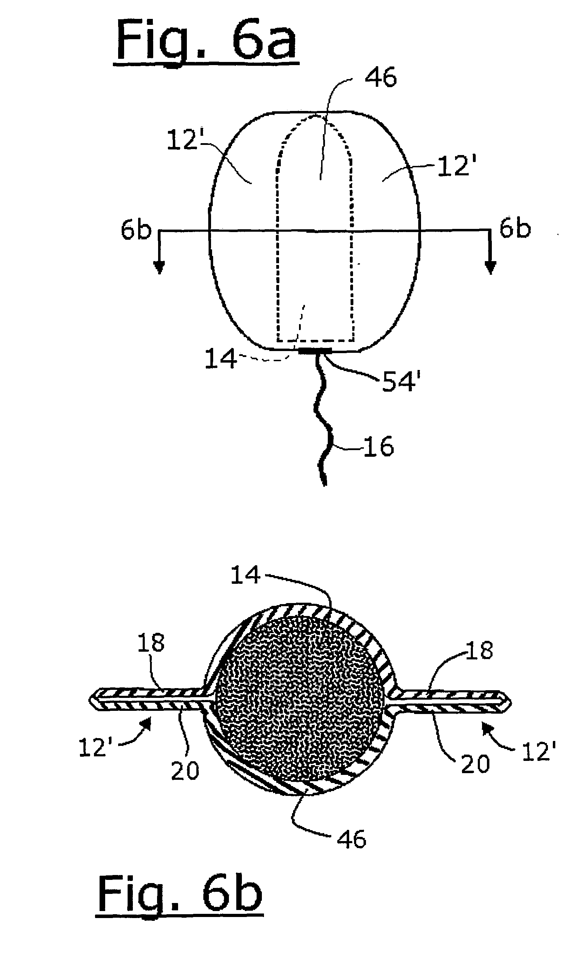

Fig. 6a shows a side elevation of an alternate embodiment of the present invention in which a cover material is bonded to itself in the form of a bag to form a fluid transport element in fluid communication with a fluid storage element.

Fig. 6b shows a cross-sectional view of the device in Fig. 6a taken along line 6b-6b.

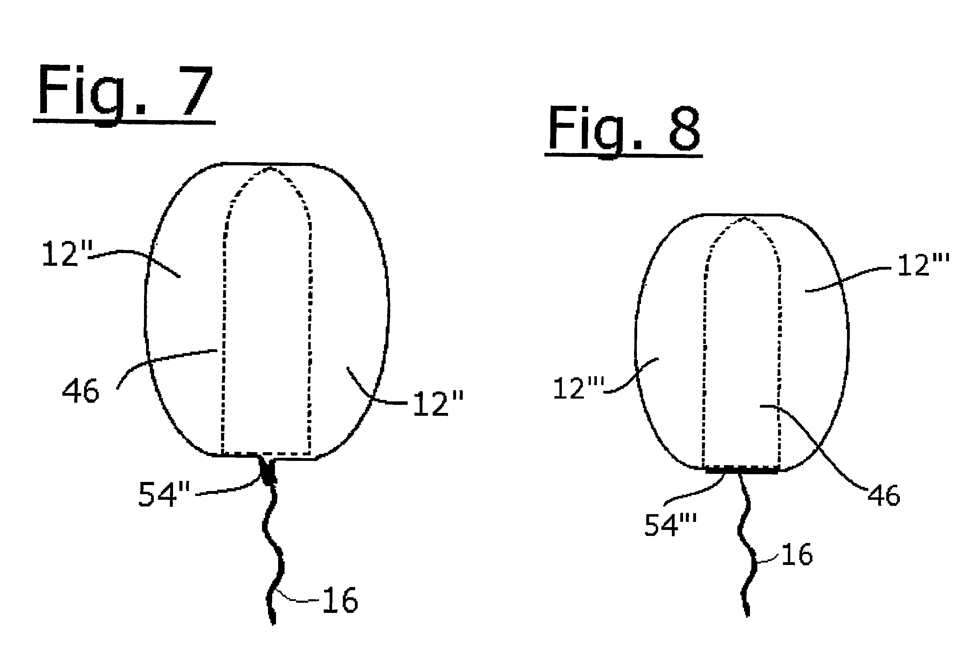

Fig. 7 shows a side elevation of an embodiment of the present invention in which the fluid transport element envelops the fluid storage element and is bonded at the withdrawal end to the withdrawal string.

Fig. 8 shows a side elevation of an embodiment of the present invention in which the fluid transport element envelops the fluid storage element and is bonded to the base of the fluid storage element.

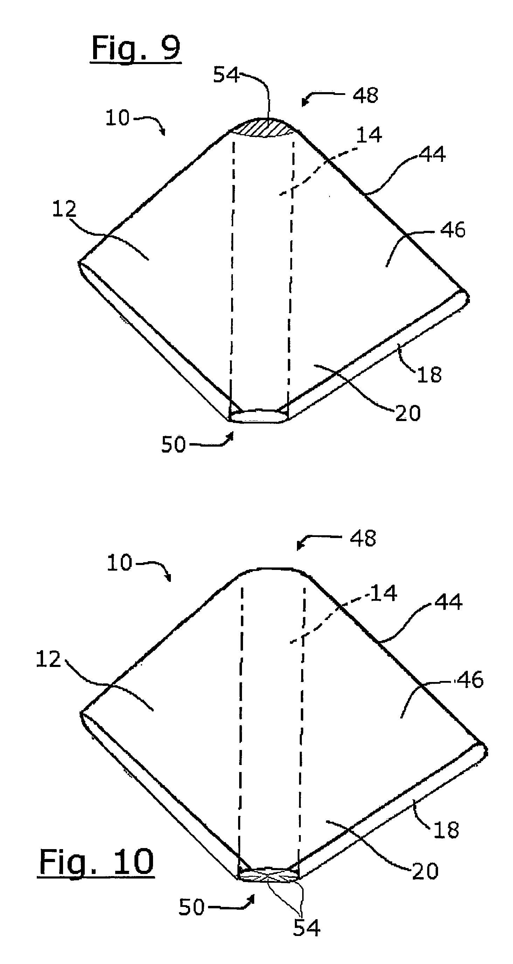

Fig. 9 shows a side elevation of an embodiment of the present invention in which the fluid transport element is attached to the insertion end of the fluid storage element.

Fig. 10 shows a side elevation of an embodiment of the present invention in which the fluid transport element is bonded to the base of the fluid storage element.

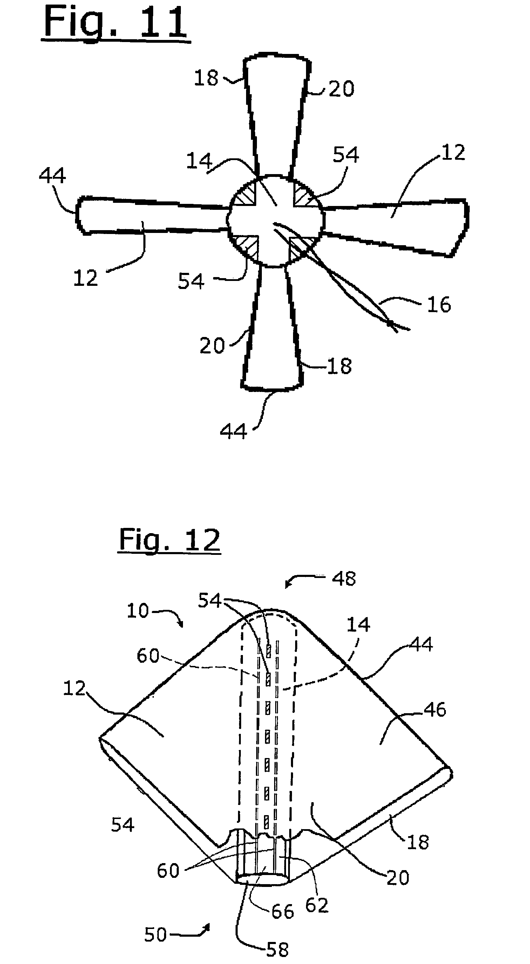

Fig. 11 shows a bottom plan view of the embodiment shown in Fig. 10.

Fig. 12 shows a side elevation of an embodiment of the present invention in which the fluid transport element is bonded to the longitudinal side of the fluid storage element in a series of aligned discrete bonds.

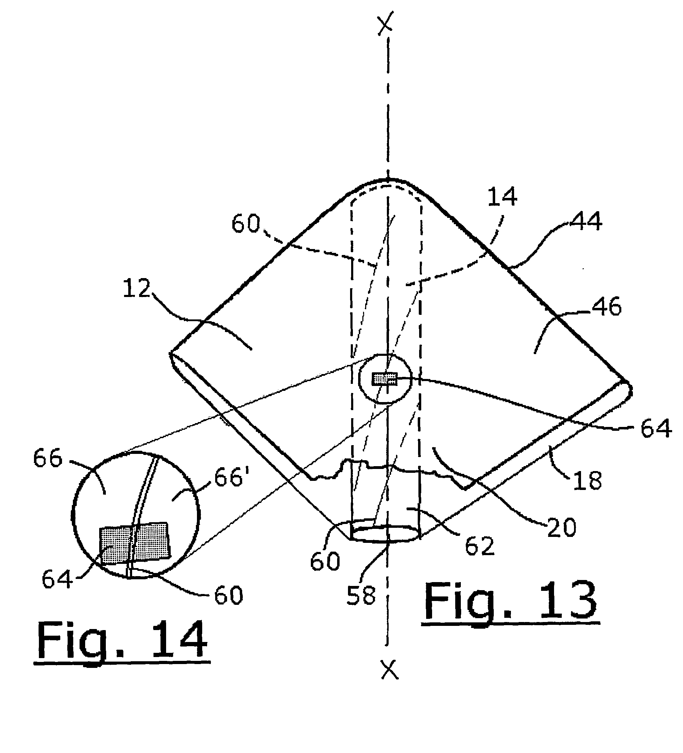

Fig. 13 shows a side elevation of an embodiment of the present invention in which the fluid transport element is bonded in at least one attachment zone having discrete spots of bonds on the longitudinal side of the fluid storage element.

Fig. 14 shows an enlarged view of a section of the embodiment shown in Fig. 13.

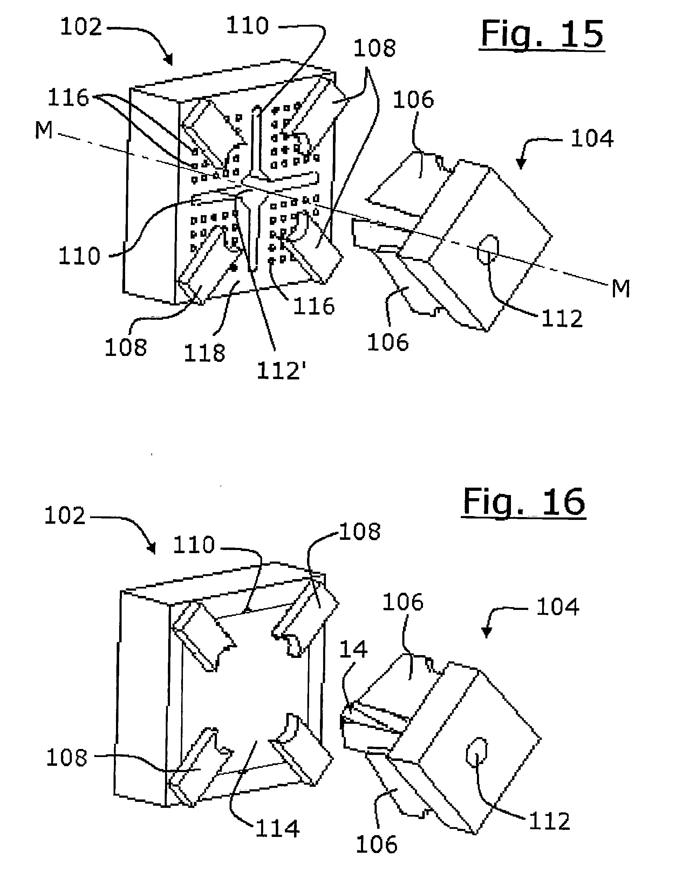

Fig. 15 shows a schematic perspective view of apparatus according to the present invention useful to manufacture an intravaginal device.

Fig. 16 shows the schematic perspective view of apparatus of Fig. 15 including a fluid storage element and a sheet of material prior to formation of the fluid transport element.

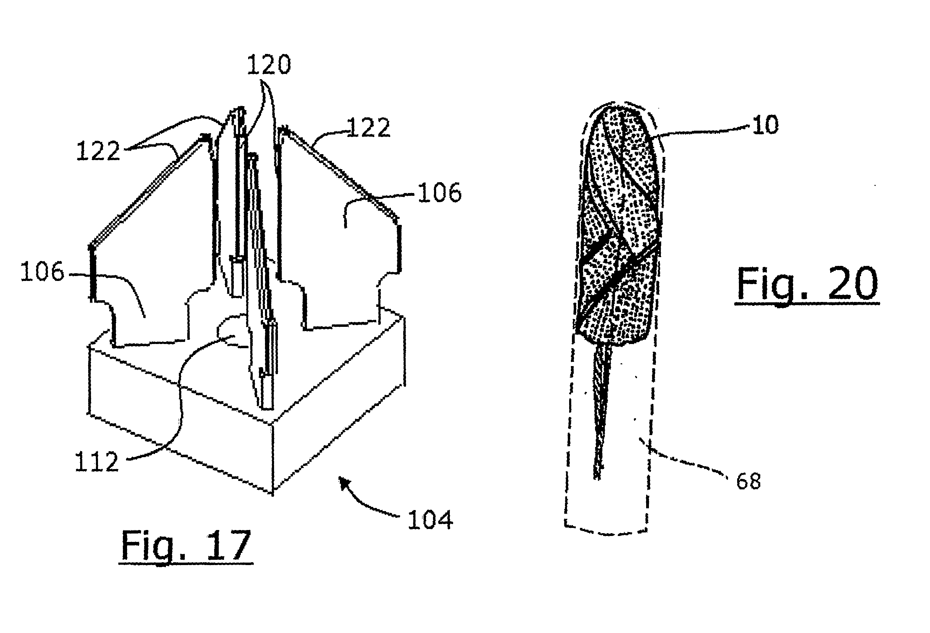

Fig. 17 shows a schematic perspective view of a male tool useful in the apparatus of Fig. 15.

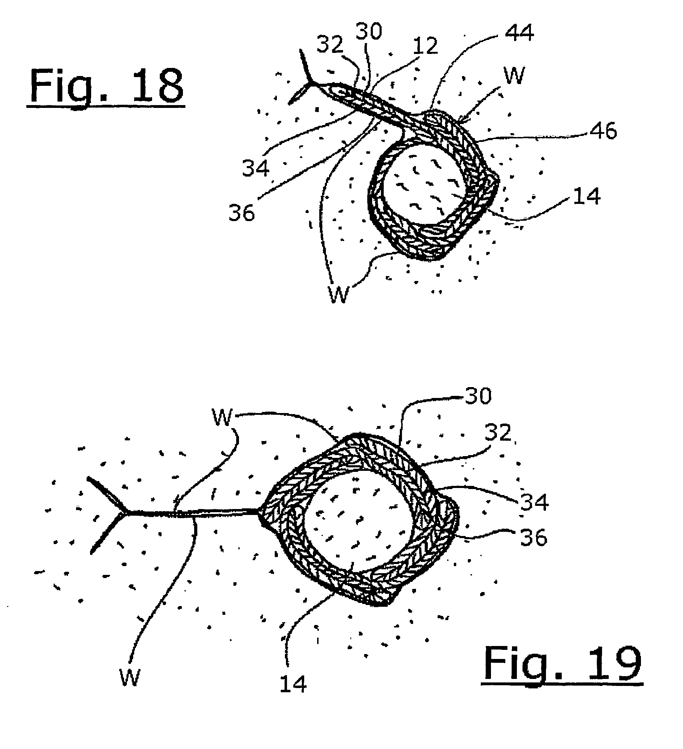

Fig. 18 shows a transverse cross-section of a human vagina with an intravaginal device according to Fig. 4b disposed therein with one fluid transport element extending away from the fluid storage element.

Fig. 19 shows a transverse cross-section of a human vagina with an intravaginal device according to Fig. 4b disposed therein with the fluid transport elements remaining wrapped around the fluid storage element.

Fig. 20 shows the device of Fig. 4 contained in an applicator device packaging element.

**Detailed Description of the Preferred Embodiments**

As used herein in the Specification and the Claims, the term "bodily fluid" and variants thereof mean bodily exudates, especially liquids that are produced by, secreted by, emanate from, and/or discharged from a human body.

As used herein in the Specification and the Claims, the term "fluids" and variants thereof relate to liquids, and especially bodily fluids.

As used herein in the Specification and the Claims, the term "sheet" and variants thereof relates to a portion of something that is thin in comparison to its length and breadth.

As used herein in the Specification and the Claims, the term "parallel plate" and variants thereof relates to a system of at least two relatively parallel sheets that are capable of moving fluids through inter-plate capillary action. The individual "plates" in the system may be flexible and/or resilient in order to move within their environment. However, they may be maintained in a substantially facing relationship with relatively constant separation at least in a localized portion of their structure (as compared with their relative length and width). Thus, two sheets could be fluted, but if the flutes were "nested", the sheets would generally remain generally parallel in any given localized portion.

As used herein in the Specification and the Claims, the term "inter-plate capillary action" and variants thereof mean the movement of fluid due to a pressure difference across a liquid-air meniscus created within a gap between two substantially parallel plates. The two plates need not be held apart a specific distance, although they should be separable to allow fluid to move between them by inter-plate capillary action. A general equation providing the rise of a fluid between parallel plates is reported as:

$$h = \frac{2\sigma * \cos\theta}{\rho * g * d}$$

in which:

  $h$ is rise of fluid between plates
  $\sigma$ is the surface tension of fluid in contact w/ plate
  $\theta$ is contact angle
  $\rho$ is density
  $d$ is distance between plates, and
  $g$ is the gravitational constant

Therefore, as long as the contact angle, $\theta$, is less than 90°, there will be some capillary attraction.

As used herein in the Specification and the Claims, the term "porous medium" and variants thereof relates to a connected 3-dimensional solid matrix with a highly ramified network of pores and pore throats in which fluids may flow.

As used herein in the Specification and the Claims, the term "separable plates" and variants thereof mean any condition of separation of the first plate and the second plate, which allows fluid to move between the plates. This includes situations in which facing surfaces of adjacent first and second plates are touching one another in portions of or across substantially all of their facing surfaces. This also includes situations in which the facing surfaces of the adjacent first and second plates are separably joined together such that upon contact with fluid, the surfaces separate enough to provide for fluid to move between them. This further includes situations in which facing surfaces of adjacent first and second plates are joined together, as long as fluid may still move freely between the surfaces.

As used herein in the Specification and the Claims, the term "in fluid communication" and variants thereof relate to elements that are arranged and configured to allow fluid to move therebetween.

As used herein in the Specification and the Claims, the term "coupled" and variants thereof relate to the relationship between two portions of an integral structure that are either portions of the same material (e.g., two portions of a folded sheet) or are materials that are joined together (e.g., two separate sheets that are bonded together).

As used herein in the Specification and the Claims, the term "fluid pervious" and variants thereof relate to a material that permits fluid or moisture to pass through without additional processing, such as aperturing. Therefore, for example, an untreated woven or nonwoven material is fluid pervious and a continuous, plastic film or metal foil is not. A nonwoven permits fluid flow via the interstices between fibers, such that fluid can flow through, either by capillary action and/or via a pressure differential from one side of the nonwoven to the other such as the pressure experienced by a tampon in use.

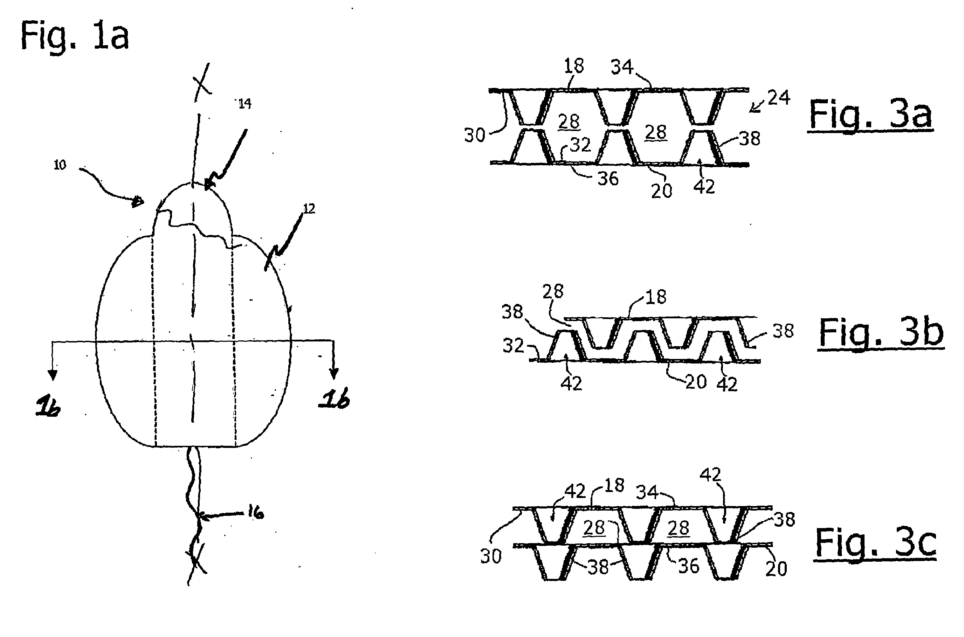

Referring to Fig. 1a-1c, this invention provides an intravaginal device 10 having at least one fluid transport element 12 in fluid communication with a fluid storage element 14 (Figs. 1a-1c show two fluid transport elements 12 located on opposite sides of the fluid storage element 14). The device may also include a withdrawal mechanism, such as a string 16.

The fluid storage element can be any convenient shape including cylindrical, cup like, hourglass, spherical, etc. It can be an absorbent or a fluid collection device. It can be in separate sections with the fluid transport element(s) bridging or connecting the sections. The fluid storage element can be made of any structure known in the art, such as compressed fibrous webs, rolled goods, foam, and the like. The material may be formed as a unitary mass or a plurality of discrete particles or agglomerations. The material may be compressed to maintain a relatively stable form, or it may be left relatively uncompressed. For example, the absorbent material may include a central portion of absorbent wood pulp material. The pulp may be covered by a thin absorbent woven or nonwoven fabric and may be coterminous with the fluff pad or completely envelop it on all sides. Absorbent materials which are uncompressed or of low density have a much higher holding capacity for fluids than high density materials. A consideration for using uncompressed materials is the bulk or volume that may be required in order to achieve sufficient absorbency.

In one preferred embodiment, the fluid storage element 14 is an absorbent tampon. Absorbent tampons are usually substantially cylindrical masses of compressed absorbent material having a central axis and a radius that defines the outer circumferential surface of the tampon. Such tampons are disclosed in e.g., Haas, US Pat. No. 1,926,900; Dostal, US Pat. No. 3,811,445; Wolff, US Pat. No. 3,422,496; Friese et al., US Pat. No. 6,310,296; Leutwyler et al., US Pat. No. 5,911,712, Truman, US Pat. No. 3,983,875; Agyapong et al., US Pat. No. 6,554,814.

Tampons also usually include a fluid-permeable cover (which may include or be replaced by another surface treatment) and a withdrawal string or other removal mechanism.

Absorbent materials useful in the formation of the absorbent body include fiber, foam, superabsorbent, hydrogels, and the like. Preferred absorbent material for the present invention includes foam and fiber. Absorbent foams may include hydrophilic foams, foams that are readily wetted by aqueous fluids as well as foams in which the cell walls that form the foam themselves absorb fluid.

Fibers may be selected from cellulosic fiber, including natural fibers (such as cotton, wood pulp, jute, and the like) and synthetic fibers (such as regenerated cellulose, cellulose nitrate, cellulose acetate, rayon, polyester, polyvinyl alcohol, polyolefin, polyamine, polyamide, polyacrylonitrile, and the like).

The fluid storage element may also be in the form of a collection cup. Examples of such devices are disclosed in Zoller, US Pat. No. 3,845,766 and Contente et al., US Pat. No. 5,295,984. Collection devices are designed to assume a normally open, concave configuration, with an open side facing a user's cervix. The collection devices may be folded, or otherwise manipulated, to facilitate insertion into the vaginal canal

The fluid transport element has at least a first plate 18 and a second plate 20. The first and second plates combine to provide a set of parallel plates, and the fluid transport elements 12 are shown as extending radially away from the fluid storage element 14. Additional plates may also be incorporated into each fluid transport element 12.

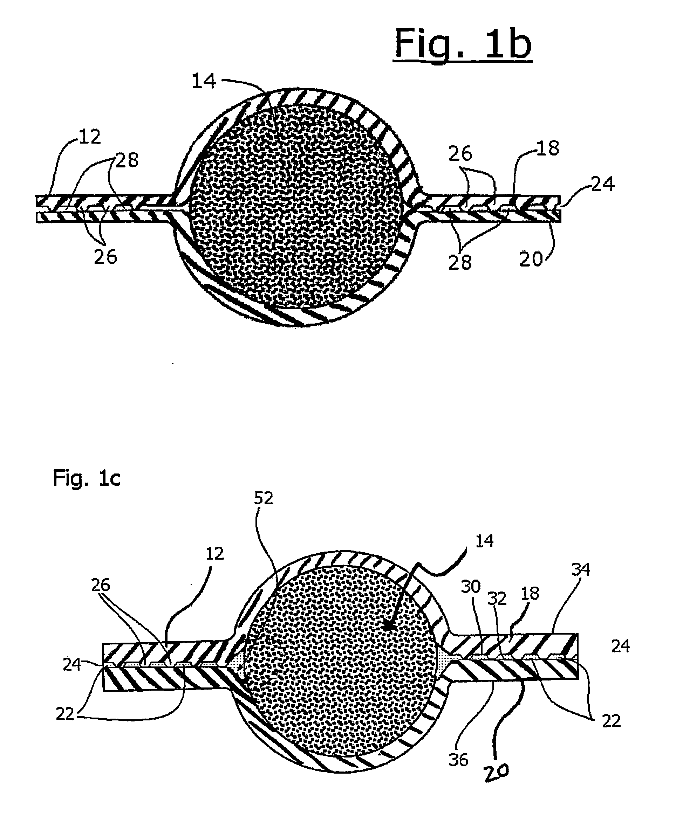

The plates are configured and arranged to allow the introduction of bodily fluid 22 to separate a plate from adjacent plate(s) (Fig. 1c). At least one opening 24 allows the introduction of bodily fluids 22. Optionally, one or more spacer elements 26 can be inserted to establish and to maintain space between adjacent plates.

Fig. 1b shows a pair of parallel plates prior to the introduction of a fluid. In this view, the facing surfaces of the adjacent plates 18, 20 are in contact. On the other hand, Fig. 1c shows the set of parallel plates separated by a bodily fluid 22, providing an inter-plate capillary gap 28 between the inwardly oriented surface 30 of the first plate 18 and the first surface 32 of the second plate 20. This inter-plate capillary gap 28 is sufficient to provide inter-plate capillary action to allow the fluid transport element 12 to acquire, to spread, and to move bodily fluids 22 from the vagina to the fluid storage element 14. The first plate 18 also has an outwardly oriented surface 34, and the second plate 20 also has an opposite surface 36.

The plates 18, 20 can be made of almost any hydrophobic or hydrophilic material, preferably sheet-like. The thickness of each plate is not critical. However, it can preferably be selected from the range of from 0.0127cm to 0.127cm (0.005 to 0.050 inch). The materials of construction and the thickness of the plates should be designed so that they are sufficiently stiff and/or resistant to wet collapse when exposed to fluid.

In particular, materials useful for forming the fluid transport element may have properties such as thermobondability to provide means to incorporate it into the intravaginal device. A representative, non-limiting list of useful materials includes polyolefins, such as polypropylene and polyethylene; polyolefin copolymers, such as ethylenevinyl acetate ("EVA"), ethylene-propylene, ethyleneacrylates, and ethylene-acrylic acid and salts thereof; halogenated polymers; polyesters and polyester copolymers; polyamides and polyamide copolymers; polyurethanes and polyurethane copolymers; polystyrenes and polystyrene copolymers; and the like. The fluid transport element may also be micro-embossed or apertured. Examples of films having apertures include for example, three-dimensional apertured films, as disclosed in Thompson, US Pat. No. 3,929,135, and Turi et al, US Pat. No. 5,567,376, as well as two-dimensional reticulated film, such as that described in Kelly, US Pat. No. 4,381,326. Figs. 2a-2c illustrate three combinations of the apertured film of Thompson.

It may be helpful to keep the exposed surface of the fluid transport element as smooth as possible. It may also be helpful to provide it with a low coefficient of friction. These characteristics may provide at least two benefits: (1) the force required to insert the intravaginal device is reduced, and (2) it reduces the damage otherwise caused by scraping of soft, tender vaginal tissue during insertion, wearing and removal. Plates 18 and 20 may be made from the same material or alternately, plate 18 may be made from a different material than plate 20.

The parallel plates can have any physical structure to provide a resistance to fluid flow vector in the direction parallel to the inwardly oriented surface 30 of the first plate 18 and the first surface 32 of the second plate 20 that is less than the resistance to fluid flow vector in the direction perpendicular to the plates. Preferably, the plates are made from any smooth material with a non-fibrous surface. Suitable materials include, without limitation, foil, waxed sheets, film, apertured film, etc. Each plate does not need to be made of the same material as its corresponding parallel plate. For instance, the first plate 18 could be an apertured film to allow fluid to enter and the second plate 20 could be a solid film to move fluid to the storage element (as shown in Fig. 2). Of course, the parallel plates should be able to transport fluid between the two layers.

The fluid transport element 12 should be strong enough to prevent rupturing during handling, insertion, and removal and to withstand vaginal pressures during use.

It is preferable that the surfaces of the fluid transport element 12 are sufficiently wettable by the bodily fluids that the intravaginal device 10 is intended to collect (this results largely from a correlation of the surface energy of the plate surface and the bodily fluid(s)). Thus, the bodily fluid will easily wet the plate, and capillarity between the plates will draw these bodily fluids from a source to a fluid storage element that is in fluid communication with the fluid transport element.

Surface treatments can be used to modify the surface energy of the plates 18, 20. In a preferred embodiment a surfactant is applied to increase the wettability of the outer or inner surfaces of the parallel plates. This will increase the rate at which the bodily fluids are drawn into and spread between a pair of plates. The surfactant can be applied uniformly to either the inner or outer surfaces or it could be applied with varying coating weights in different regions.

A useful measure to determine the wettability of a plate surface is its contact angle with 1.0 % saline. Preferably, the contact angle with 1.0% saline is less than about 90 degrees.

In order to accomplish this, the materials of plates can be chosen from those materials that are known in the art to have low energy surfaces. It is also possible and useful to coat materials that have high-energy surfaces with a surface additive, such as a non-ionic surfactant (e.g., ethoxylates), a diol, or mixtures thereof, in order to increase their wettability by bodily fluids. Such additives are well known in the art, and examples include those described in Yang et al., US App. No. 2002-0123731-A1, and US Pat. No. 6,570,055. Other means of increasing wettability can also be used, such as by corona discharge treatment of, for example, polyethylene or polypropylene, or by caustic etching of, for example, polyester.

The parallel plates forming the fluid transport element can be of any flexibility as long as the material is able to transport fluid to the fluid storage element while the device is in use. It is also preferable that the fluid transport element be sufficiently flexible to provide the user with comfort while inserting, wearing, and removing the device.

The surfaces of the first and second plates facing each other can have a variety of surface textures, ranging from smooth to highly textured. The texturing element may be included as a spacer 26.

The value of spacers 26 or texture may be based on the material's ability to withstand wet collapse when simultaneously subjected to compressive forces and fluid.

The spacer elements 26 can be separate elements applied to one or more of the plates, or they can be integral portions of a plate that extend away from one of the plate's major surfaces. A representative list of such separate spacer elements includes, without limitation, foamed materials such as polystyrene foam; particles such as beads and crystals; discontinuous material such as netting, thread, wax, adhesive, any discrete element that causes a separation between the plates and the like.

Integral spacer elements can be thickened portions of the plate material or deformations of the plate material. A representative list of such an integral spacer element includes, without limitation, nubbles, embossments, corrugations, deformations, and the like. Included in this definition are surface treatments that permanently bond a secondary material to a surface of a first. One example of a deformation is provided as the sidewalls 38 of a "three-dimensional" polymeric apertured formed film material shown in Figs. 3a-3c. First and second plates 18, 20 made from apertured formed film with the sidewalls 38 facing each other as the inward surface 30 of the first plate 18 and the first surface 32 of the second plate 20 can be used to increase the texture of the plates. While not wishing to be held to this theory, it is believed that the texturing reduces the viscosity of the fluid being transported. The texture can also be in a gradient. For example, in one embodiment, the texture of the plates has a gradient from smooth near the edge of the plates where the fluid enters the fluid transport element to more textured where the fluid is absorbed.

Referring again to Fig. 2, the spacer elements may be formed as nubbles 40 extending from the inward surface 30 of the first plate 18 and resting on the first surface 32 of the second plate 20.

In order to maintain stability against sliding of the plates with respect to each other and changing of the space between them, it is acceptable, and may be preferable, to secure some local areas of contact between the spacer elements 26 and the adjacent plate or even between spacer elements 26 of two adjacent plates. The plates may be secured through means known to those of ordinary skill in the art. A representative list of such securing means includes, without limitation, thermobonding, adhering, crimping, embossing, ultrasonic bonding or welding, and the like. The adhesive may be applied between the spacer elements and the first and second plates. Preferably, the adhesive is wettable.

The at least one opening can be at the edge of the plates, e.g., edges of adjacent plates are separated, or plates themselves may have at least one opening. The openings need not be uniform. For example, one opening may be located at the edge of the plates and a plurality of smaller openings or apertures can be distributed throughout one or more plate. Preferably, each plate has a plurality of openings distributed throughout. An example of openings distributed throughout is an apertured film. The distribution can be uniform or arranged to provide regions of higher open area and regions of lower open area.

A plurality of openings or apertures 42 may extend through at least one of the first and second plates 18, 20. These apertures 42 may extend completely through the plate and may be present in both of the plates. The apertures 42 allow fluid that contacts the outward surface 34 of the first plate 18 or the opposite surface 36 of the second plate 20 to flow into the inter-plate capillary gap 28 between the plates with as little restriction as possible. In the example of apertured film, it is preferred that the total surface area of the plate occupied by the openings is from 5% to preferably 50%. More preferably, it will be from 25% to 45%. Having this much open area formed in a plate will allow fluid that is deposited on that plate to easily flow into the inter-plate capillary gap 28.

It is preferable that any individual opening (e.g., edge opening 24 of fluid transport element 12 or aperture 42) is large enough to easily pass any highly viscous material, including menstrual fluid. While the geometry of the openings is not critical, the openings 24, 42 should be sized sufficient to allow easy passage of non-absorbable material. If the apertures 42 are not circular, then the measurement should be made across the narrowest part of the opening, which would be most restrictive to the flow of non-absorbable material.

In the example of unapertured film that has an opening 24 at the ends of the plates 18, 20, the size of the opening 24 is a result of the fluid's ability to separate the plates.

It is preferred that the apertures 42 are large enough to let viscous fluid pass through but not too large to create too rough of a surface as to compromise the wearer's comfort. A preferred aperture 42 is circular and is between 0.0254 cm and 0.1016 cm (10 mils and 40 mils) in diameter. Most preferably it is between 0.04572 cm and 0.06858 cm (18 mils and 27 mils).

Open area may be determined by using image analysis to measure the relative percentages of apertured and unapertured, or land, areas. Essentially image analysis converts an optical image from a light microscope into an electronic signal suitable for processing. An electronic beam scans the image, line-by-line. As each line is scanned, an output signal changes according to illumination. White areas produce a relatively high voltage and black areas a relatively low voltage. An image of the apertured formed film is produced and, in that image, the holes are white, while the solid areas of thermoplastic material are at various levels of gray. The more dense the solid area, the darker the gray area produced. Each line of the image that is measured is divided into sampling points or pixels. The following equipment can be used to carry out the analysis described above: a Quantimet Q520 Image Analyzer (with v. 5.02B software and Grey Store Option), sold by LBICA/Cambridge Instruments Ltd., in conjunction with an Olympus SZH Microscope with a transmitted light base, a plan 1.0x objective, and a 2.50x eyepiece. The image can be produced with a DAGE MTI CCD72 video camera.

A representative piece of each material to be analyzed is placed on the microscope stage and sharply imaged on the video screen at a microscope zoom setting of 10x. The open area is determined from field measurements of representative areas. The Quantimet program output reports mean value and standard deviation for each sample.

Referring for example, to Figs. 4 and 5, the first and second plates 18, 20 may be extensions of the same sheet-like material, e.g., formed by a fold in a sheet of material (as shown in Figs. 4a-4c), or they may be separate elements (i.e., adjacent to each other but not necessarily joined). In a folded embodiment, the material is preferably folded to form a pleat with the first and second plates facing each other.

A preferred embodiment with pleats is shown in Figs. 4a-4e, where the pleats 44 are folds in the cover material 46. The pleats 44 create plates that are bendable about an infinite number of bending axes ($b_{1-i}$-$b_{1-i}$) that are substantially parallel to the longitudinal axis (X-X) of the product, which longitudinal axis extends through the insertion end 48 and withdrawal end 50. These bending axes allow the plates to wrap around the product, either partially or completely. One such bending axis ($b_1$-$b_1$) is shown in Fig. 4a.

The fluid transport element 12 is in fluid communication with the fluid storage element 14 and directs fluid from the vagina to the storage element 14. Generally, fluid will be directed from each fluid transport element 12 to a particular region of the fluid storage element associated with that fluid transport element. Thus, if the device has only one fluid transport element 12, the fluid will contact the fluid storage element in one interface 52.

Therefore, additional fluid transport elements 12 directing fluid to additional locations of the fluid storage element 14 will improve the efficient usage of the fluid storage element 14. For example, two fluid transport elements 12 could be directed to opposite sides of the fluid storage element 14, as shown in Figs. 1a-1c. Each additional fluid storage element 12 can direct fluid to additional interface locations 52 of the fluid storage element 14. For example, four evenly spaced fluid transport elements 12 allow fluid to be directed to each quarter of the fluid storage element 14 surface as shown in Figs 4a-e. Five or more elements would provide even more direct access. This can allow the fluid to contact the fluid storage element 14 uniformly and help to prevent or reduce local saturation of the fluid storage element 14.

While the above description provides for direct fluid communication between a fluid transport element 12 and the fluid storage element 14, direct fluid contact is not necessary. There can be fluid communication through an intermediate element, such as a porous medium (e.g., a foam or fibrous structure), a hollow tube, and the like.

Enlarging the area of the interface 52 between the fluid transport element 12 and fluid storage element 14 can also help to maximize the fluid communication. For example, elongating the interface by increasing the length of the fluid transport element 12 allows more fluid to flow into the fluid storage element 14.

The fluid transport element 12 may extend in any orientation from the surface of the fluid storage element 14. It is not necessary for the fluid transport element to be on the surface of the fluid storage element.

The inter-plate capillary gap 28 formed by first plate 18 and second plate 20 can terminate at the interface 52 or can extend into and/or through the fluid storage element 14. An example of the fluid transport element 12 extending into the fluid storage element 14 is shown in Fig. 5. The first and second plates can have additional layers on top of them as long as these additional layers allow fluid to enter the plates. The first and second plates can end at the boundary of the transport element or can extend into the fluid storage element 14.

The fluid transport element 12 may be formed to extend from the surface of the fluid storage element 14 as in Figs. 1a-1c. It can be made in any convenient shape, including semicircular, triangular, square, hourglass etc. Additionally the two plates of the element do not have to be completely coextensive, as long as they are at least partially in a facing relationship.

Parallel plates can be held in close proximity to the storage element in a variety of ways including directly or indirectly via an additional element to the storage element. A variety of methods can be used to attach the fluid transport element 12 including but not limited to heat, adhesive, ultrasonic, sewing, and mechanically engaging the fluid storage element 14. An example of a heat-bonded attachment 54 is shown in Fig. 4a.

The fluid transport element(s) 12 can be attached at the sides, insertion end 48, and/or withdrawal end 50 of the intravaginal device. Additionally, the fluid transport element(s) 12 may be attached to themselves and not to the storage element as in a relatively loose bag covering of the storage element. The fluid transport element(s) 12 could also be attached to the withdrawal string.

The fluid transport element may be attached directly to the fluid storage element or may be attached to itself in one or more locations. Such attachment or adherence to itself or to the fluid storage element may be by any known means, including, for example, adhesive, ultrasonic, co-embossing, thermobonding, mechanical bonding (such as crimping), and the like. In one embodiment, the fluid transport element is formed of a material that is capable of being thermobonded. Alternately, the material may formed of two different materials having different melting points, at least one of which would also be capable of thermobonding.

In an embodiment shown in Figs. 6a and 6b, the cover material 46 substantially envelops the fluid storage element 14 (shown as a tampon), forming a bag or sack structure 56. This structure provides a pair of fluid transport elements 12' formed by portions of the cover material 46. In this embodiment, the cover material 46 is draped over the insertion end 48 of the tampon with the edges of the material brought together about the withdrawal end 50 and then bonded to itself 54'. The resulting fluid transport element 12' can then be folded around the tampon in the manner shown in Fig. 4b.

Other embodiments similar to that shown in Fig. 6 are possible. For example, Fig. 7 shows the attachment 54" of the fluid transport element 12 to the withdrawal string 16, and Fig. 8 shows the attachment 54''' at the withdrawal end 50, especially to the base 58 of the fluid storage element 14 (the base 58 being the generally circular surface from which the withdrawal string 16 may extend). In all of these embodiments, the cover material 46 and the associated fluid transport element 12 substantially envelop the fluid storage element 14 but do not significantly affect the performance of the fluid storage element 14. For example, if the fluid storage element 14 had been compressed and expands upon exposure to fluid, the expansion of the fluid storage element 14 would not be affected or inhibited by the attachment or bonding of the fluid transport element 12 to the fluid storage element 14.

In the embodiments described and shown in Figs. 6-8, it is not necessary for the fluid storage element 14 to be a unitary element. For example, the fluid storage element 14 may have multiple distinct portions or segments. The segments may be attached together or may be discrete. Examples of discrete segments may be relatively loose absorbent material or compressed cellulosic tablets. However, these discrete segments could be at least partially contained to permit the fluid transport element 12 to form parallel plates, as described above.

In an alternate embodiment of the invention shown in Fig. 9, the fluid transport element 12 and the fluid storage element 14 have an attachment 54 at the insertion end 48 of fluid storage element 14. Pleats 44 formed in the fluid transport element 12 may be folded around the tampon as previously shown in Fig. 4b. Additionally, the lower portions 60 of the sheet material may also be attached to withdrawal end 50 of the fluid storage element 14, as described above and below, to prevent inversion of the fluid transport element 12 upon withdrawal.

In embodiments where the fluid transport element 12 is bonded or gathered at the withdrawal end 50 of the fluid storage element 14, it is preferable to minimize bunching of the fluid transport element 12 material to limit interference during insertion and withdrawal of the device.

Although not required, the sheet material used to form the fluid transport element 12 may initially be in a shape such that the sheet has at least one corner. The sheet material is placed over the fluid storage element 14 such that at least one portion of the sheet extends away from the fluid storage element 14. In one embodiment, the sheet has a plurality of corners, and each corner may be attached to the withdrawal end 50 of the fluid storage element 14. For example, if four sets of parallel plates are desired, the sheet material may be a square.

If the fluid storage element 14 is a compressed tampon having embossed grooves such as those disclosed in US Pat. No. 5,165,152, the attachment may be on the outer most surface (non-embossed) or in the grooves. Attachment may take place before, during, and/or after fluid storage element 14 compression.

The embodiment of Figs. 10 and 11 is similar to that of Fig. 9. In particular, the corners of the fluid transport element 12 are attached to the base 58 of the fluid storage element 14. As seen in Fig. 11, the corners preferably do not overlap the center of the circular base 58.

When a compressed tampon having grooves 60 is used as the fluid storage element 14, it is likely that the tampon performs optimally if permitted to expand without restriction by the fluid transport element. While some compressed tampons expand due to dry expansion, others expand when exposed to fluid. One example of such a compressed tampon having grooves is the o.b.® tampon available from McNEIL-PPC, Inc., Skillman, NJ.

In the embodiments shown in Figs. 12-14, the fluid storage element 14 is a compressed tampon having an exterior surface 62 and grooves 60. Grooves 60 have an interior portion, which becomes part of the exterior surface 62 of the tampon upon absorption of fluids and the resultant tampon expansion. Because the fluid transport element 12 is attached to the exterior surface 62 of the tampon at its withdrawal end 50, it does not extend into the tampon grooves 60. Thus, the fluid storage element 14 may expand without any interference from the fluid transport element 12. In other words, the fluid transport element 12 does not significantly limit the functionality of the fluid storage element 14. Pleats 44' form in the fluid transport element 12 and may be similarly folded around the tampon as previously shown in Fig. 4b.

As shown in Fig. 12, a tampon having straight grooves is attached to the fluid transport element 12 using a series of heat bonds 54 along one or more single line(s) along the tampon. This provides easier alignment of the attachment 54 and the exterior surface 62 of the tampon as the bond line may be registered accurately to avoid coinciding with the grooves 60. Thus, the fluid transport element 12 may be readily attached along the longitudinal side without interfering with the expansion of the tampon.

In a similar embodiment shown in Figs. 13 and 14, the fluid transport element 12 may be attached along the longitudinal side of a tampon having spirally oriented grooves. In this embodiment an attachment zone 64 of fluid transport element 12 extends from one lobe 66 and across groove 60 to adjacent lobe 66'. As previously described, materials such as apertured films have a certain amount of elasticity and may be designed to permit the tampon expansion, especially the material located within the interior portion of the grooves 60.

If desired, the attachment zone 64 may be oriented in any direction relative to the longitudinal axis X-X of the fluid storage element 14. As shown in Figs. 13 and 14, the attachment zone 64 comprises a matrix or other grouping of discrete bonds, such as dots or spots. This allows the interface between the fluid transport element 12 and the fluid storage element 14 to remain as open to fluid flow as possible.

As previously mentioned and shown, the fluid transport element 12 may be attached to the fluid storage element 14 be any number of methods and embodiments. For example and with reference to Figs. 15-17, a tampon may be manufactured as shown in Friese, US Pat. No. 4,816,100, and either Friese et al., US Pat. No. 6,310,269, or Leutwyler et al., US Pat. No. 5,911,712. However, after the tampon is formed and prior to packaging, an additional process employing a forming tool 102, a male tool 104 having a plurality of blades 106, and thermobonding elements 108 applies a fluid transport element 12 to the fluid storage element 14. The tools are aligned in a manner that the blades 106 of the male tool 104 cooperate with corresponding slots 110 in the forming tool 102. In addition, each of the tools has a central aperture 112, 112' through which a fluid storage element 14 may pass during processing.

In somewhat more detail, an individual sheet 114 of material is separated from a supply (not shown) and placed on the forming tool 102. A vacuum is drawn across the forming tool 102 via a plurality of vacuum ports 116 on the face 118 of the forming tool 102 to hold the individual sheet 114 in place.

The blades 106 of the male tool 104 are shown arranged radially about the central aperture 112 in the male tool 104 (as shown in Fig. 17). The blades 106 cooperate to hold the fluid storage element 14 in line with the central aperture 112. A pushrod (not shown) is arranged to penetrate the central aperture 112 of the male tool 104 and to bear on the base of the fluid storage element 14. In the preferred embodiment shown in Figs. 15-17, four blades 106 are arranged at equal angles about the central aperture 112. Each blade 106 provides a guide edge 120 facing the fluid storage element 14 (when present) and a pleating edge 122 disposed radially outwards from the guide edge 120. The pleating edge 122 may be an edge that is adjacent the guide edge 120, or it may be separated by one or ore intermediate portions of the blade 106.

In operation, the male tool 104 holding a fluid storage element 14 is moved along the machine axis (M-M) aligned with the central apertures 112, 112' toward the forming tool 102 carrying the individual sheet 114. The insertion end 48 of the fluid storage element 14 contacts the individual sheet 114 and urges it through the central aperture 112' of the forming tool 102. The pleating edges 112 of the blades 106 urge corresponding portions of the individual sheet 114 through the slots 110 of the forming tool 102 creating four sets of parallel plates 18, 20.

Once the fluid storage element 14 is inserted into the central aperture 112' of the forming tool 102 with only a portion of the withdrawal end 50 remaining exposed, thermobonding elements 108 extend into the space between the blades 106 to bond the four corners of the individual sheet 110 to the exterior surface 62 of the fluid storage element 14, forming the fluid transport element 12. The pushrod may then continue to move the insertable device 10 into and through the central aperture 112' of the forming tool 102. The fluid transport element 12 may then be folded about the fluid storage element 14. The resulting insertable device may then be packaged in a hygienic overwrap as is well known in the art.

While the process described above in reference to Figs. 15-17 employs blades 106 that have a guide edge 120 that is shorter than the fluid storage element 14, this relationship may be altered. For example, the blades 106 could be modified to have a guide edge 120 that is longer than the fluid storage element 14 or the system could otherwise be modified to allow the leading portions 124 to contact the individual sheet 114, first. This permits the formation of a small gap between the insertion end 48 of the tampon and the individual sheet 114 that may allow more free expansion of the tampon without restriction by the fluid transport element 14 during use.

During use, fluid transport element(s) 12 can take on many configurations within the vagina. For example, a fluid transport element 12 may extend into the vagina away from the fluid storage element 14, as shown in Fig. 18. Alternatively, and the fluid transport element(s) 12 may remain wound about the fluid storage element 14, contacting the vaginal wall "W" only through the first surface 30 (Fig. 19).

A withdrawal mechanism, such as withdrawal string 16, is preferably joined to the intravaginal device 10 for removal after use. The withdrawal mechanism is preferably joined to at least the fluid storage element 14 and extends beyond at least its withdrawal end 50. Any of the withdrawal strings currently known in the art may be used as a suitable withdrawal mechanism, including without limitation, braided (or twisted) cord, yarn, etc. In addition, the withdrawal mechanism can take on other forms such as a ribbon, loop, tab, or the like (including combinations of currently used mechanisms and these other forms). For example, several ribbons may be twisted or braided to provide parallel plates structures.

Tampons are generally categorized in two classes: applicator tampons and digital tampons, and a certain amount of dimensional stability is useful for each type of tampon. Applicator tampons use a relatively rigid device to contain and protect the tampon prior to use. To insert the tampon into a body cavity, the applicator containing the tampon is partially inserted into the body cavity, and the tampon can be expelled from the applicator into the body cavity. In contrast, digital tampons do not have an applicator to help guide them into the body cavity and require sufficient column strength to allow insertion without using an applicator.

While the applicator tampon is protected by the rigid applicator device and the applicator tampon need not as have as high a degree of column strength as a digital tampon, applicator tampons do require dimensional stability (especially radial) to be acceptable for use. This dimensional stability provides assurance, for example, that the tampon will not prematurely grow and split its packaging material or become wedged in a tampon applicator.

Further, the intravaginal device can be collapsed for packaging and insertion. For example, at least a portion of a major surface of the fluid transport element 12, such as the first surface 30, may be in contact with at least a portion of an outer surface of the fluid storage element 14. This can be achieved by wrapping the fluid transport element(s) around the fluid storage element 14 (as shown in Fig. 4b). Alternatively, the fluid transport element(s) 12 may be folded or pleated (e.g., in an accordion-like manner) against the fluid storage element 14. The thus-compacted device can then be packaged, (e.g., within an applicator or alone in a wrapper). Fig. 20 shows a wrapped tampon within an applicator 68 (in phantom).

The specification and embodiments above are presented to aid in the complete and non-limiting understanding of the invention disclosed herein. Since many variations and embodiments of the invention can be made without departing from its spirit and scope, the invention resides in the claims hereinafter appended.

## Claims

1. A fluid management device (10) for use in a mammalian body, the device comprising:

   a) a fluid storage element (14) having a longitudinal axis (x-x), an insertion end (48) and a withdrawal end (50); and
   b) at least one fluid transport element (12) being in fluid communication with the fluid storage element (14), the at least one fluid transport element (12) comprising:

   (i) a first plate (18) having an outwardly oriented surface (34) and an inwardly oriented surface (30);
   (ii) a second plate (20) coupled to the first plate (18); that has a first surface (32) disposed and maintained in facing relationship with the inwardly oriented surface (30) of the first plate (18) and an opposite surface (36); and that is capable of separating from the first plate (18) sufficiently to provide inter-plate capillary action (28);

   wherein the at least one fluid transport element is bendable about an axis substantially parallel to the longitudinal axis (x-x) of the fluid storage element.

2. The device of claim 1 wherein the fluid transport element (12) comprises an apertured film.

3. The device according to claim 1 or claim 2, wherein the fluid transport element (12) is attached (54) to the withdrawal end (50) of the fluid storage element (14).

**4.** The device of claim 3 wherein the fluid element (12) is attached to the base of the fluid storage element (14).

**5.** The device according to claim 1 or claim 2, wherein the fluid storage element (14) has at least one longitudinal side between the insertion (48) and withdrawal ends (50); and the fluid transport element (12) is attached on the at least one longitudinal side of the fluid storage element (14).

**6.** The device according to claim 1 or claim 2, wherein the fluid storage element (14) has at least one longitudinal side between the insertion (48) and withdrawal ends (50); and the fluid transport element (12) is attached to itself proximate the withdrawal end (50) of the fluid storage element (14).

**7.** The device according to claim 1 or claim 2, wherein the fluid storage element (14) has at least one longitudinal side between the insertion (48) and withdrawal ends (50); and wherein the fluid transport element is attached to a withdrawal string (16) to substantially envelop the fluid storage element (14).

**8.** The device of claims 5, 6 or 7, wherein the fluid transport element (12) is attached at the withdrawal end (50) of the fluid storage element (14).

**9.** A method of producing an intravaginal device (10) comprising the steps of:

a) separating an individual sheet (114) from a supply of material having properties useful to move bodily fluids;
b) providing a fluid storage element (14) having a longitudinal axis (x-x), longitudinal sides, an insertion end (48) and a withdrawal end (50);
c) engaging the individual sheet (114) with the insertion end (48) of the fluid storage element (14) and with pleating edges (44) of forming blades;
d) urging the individual sheet (114) through a forming tool (102) with relative movement of the forming tool (102) in relation to the fluid storage element (14) and the pleating edges (44) of forming blades;
e) bonding at least a portion of the individual sheet (114) to the fluid storage element (14) to form at least a portion of the individual sheet (114) into at least one fluid transport element (12) capable of extending radially away from the fluid storage element (14);
f) folding the at least one fluid transport element (12) about an axis parallel to longitudinal axis (x-x) of the fluid storage element (14); and
g) packaging the resultant intravaginal device (10).

**10.** Apparatus for producing an intravaginal device (10) comprising:

a) a forming tool (102) comprising:

(i) a holding plate having a plurality of vacuum ports (116) formed in a face (118) thereof;
(ii) a substantially circular aperture disposed on the plate having a plurality of slots (110) connected to and extending therefrom;

b) a male tool (104) comprising a plurality of forming blades (106), each blade having a guide edge, arranged radially about an aperture (112);
c) at least one bonding element (108) moveable toward the aperture (112) in the forming tool (102); and
d) at least one pushrod moveable through the apertures (112) of each of the forming tool (102) and the male tool (104);

wherein the apertures (112) of the forming tool (102) and male tool (104) are aligned along a machine axis to permit the pushrod to move fluid storage element (14) through each aperture (112), the forming blades (106) of the male tool (104) are aligned with the slots (110) of the forming tool (102), and the guide edges of the forming blades (106) are arranged and configured to accommodate a fluid storage element (14) aligned with the aperture (112) of the male tool (104).

**11.** Apparatus of claim 10 wherein each forming blade (106) has a pleating edge (112') engageable with a sheet (114) held on the face (118) of the holding plate.

**12.** Apparatus of claim 11, wherein the pleating edge (112') is adjacent the guide edge.

**13.** Apparatus of claim 11, wherein the pleating edge (112') is separated from the guide edge by at least one intermediate portion of the forming blade (106).

**14.** Apparatus of claim 10, wherein the at least one bonding element (108) is moveable in a plane parallel to the face of the holding plate (118).

**15.** Apparatus of claim 14, wherein the at least one bonding element (108) is moveable to extend into a space between adjacent forming blades (106) when the forming tool (102) and male tools (104) are in an engaged position.

**16.** Apparatus of claim 10, wherein the at least one bonding element (108) is a thermobonding element.

**17.** Apparatus of claim 10, wherein the fluid storage element (14) has a length, and the guide edge of each forming blade (106) has a length that is not greater than the length of the fluid storage element (14).

**18.** Apparatus of claim 10, wherein the fluid storage element (14) has a length, and the guide edge of each forming blade (106) has a length that is greater than the length of the fluid storage element (14).

**Patentansprüche**

**1.** Flüssigkeitsverwaltungsvorrichtung (10) zur Verwendung in einem Säugetierkörper, wobei die Vorrichtung Folgendes umfasst:

a) ein Flüssigkeitsspeicherelement (14) mit einer Längsachse (x-x), einem Einführende (48) und einem Entnahmeende (50); und
b) mindestens ein Flüssigkeitstransportelement (12) in Flüssigkeitsverbindung mit dem Flüssigkeitsspeicherelement (14), wobei das mindestens eine Flüssigkeitstransportelement (12) Folgendes umfasst:

(i) eine erste Platte (18) mit einer nach außen orientierten Oberfläche (34) und einer nach innen orientierten Oberfläche (30);
(ii) eine zweite Platte (20), die an die erste Platte (18) gekoppelt ist; die eine erste Oberfläche (32), die der nach innen orientierten Oberfläche (30) der ersten Platte (18) zugewandt angeordnet und gehalten ist, und eine gegenüberliegende Oberfläche (36) aufweist; und die in der Lage ist, sich von der ersten Platte (18) ausreichend zu trennen, um eine Zwischenplattenkapillarwirkung (28) bereitzustellen;

wobei das mindestens eine Flüssigkeitstransportelement um eine Achse biegbar ist, die im Wesentlichen parallel zu der Längsachse (x-x) des Flüssigkeitsspeicherelements verläuft.

**2.** Vorrichtung nach Anspruch 1, wobei das Flüssigkeitstransportelement (12) eine perforierte Folie umfasst.

**3.** Vorrichtung nach Anspruch 1 oder 2, wobei das Flüssigkeitstransportelement (12) an dem Entnahmeende (50) des Flüssigkeitsspeicherelements (14) befestigt ist (54).

**4.** Vorrichtung nach Anspruch 3, wobei das Flüssigkeitselement (12) an der Basis des Flüssigkeitsspeicherelements (14) befestigt ist.

**5.** Vorrichtung nach Anspruch 1 oder 2, wobei das Flüssigkeitsspeicherelement (14) mindestens eine Längsseite zwischen dem Einführ- (48) und dem Entnahmeende (50) aufweist; und das Flüssigkeitstransportelement (12) an der mindestens einen Längsseite des Flüssigkeitsspeicherelements (14) befestigt ist.

**6.** Vorrichtung nach Anspruch 1 oder 2, wobei das Flüssigkeitsspeicherelement (14) mindestens eine Längsseite zwischen dem Einführ- (48) und dem Entnahmeende (50) aufweist; und das Flüssigkeitstransportelement (12) benachbart zum Entnahmeende (50) des Flüssigkeitsspeicherelements (14) an sich selbst befestigt ist.

**7.** Vorrichtung nach Anspruch 1 oder 2, wobei das Flüssigkeitsspeicherelement (14) mindestens eine Längsseite zwischen dem Einführ- (48) und dem Entnahmeende (50) aufweist; und wobei das Flüssigkeitstransportelement an einem Entnahmefaden (16) befestigt ist, um im Wesentlichen das Flüssigkeitsspeicherelement (14) zu umhüllen.

**8.** Vorrichtung nach einem der Ansprüche 5, 6 und 7, wobei das Flüssigkeitstransportelement (12) an dem Entnahmeende (50) des Flüssigkeitsspeicherelements (14) befestigt ist.

**9.** Herstellungsverfahren für eine intravaginalen Vorrichtung (10), das die folgenden Schritte umfasst:

a) Trennen eines einzelnen Blatts (114) aus einer Materialversorgung mit Eigenschaften, die beim Bewegen von Körperflüssigkeiten nützlich sind;
b) Bereitstellen eines Flüssigkeitsspeicherelements (14) mit einer Längsachse (x-x), Längsseiten, einem Einführende (48) und einem Entnahmeende (50);
c) Greifen des einzelnen Blatts (114) mit dem Einführende (48) des Flüssigkeitsspeicherelements (14) und mit Faltkanten (44) von Formklingen;
d) Drängen des einzelnen Blatts (114) durch ein Formwerkzeug (102) mit einer relativen Bewegung des Formwerkzeugs (102) in Relation zu dem Flüssigkeitsspeicherelement (14) und den Faltkanten (44) der Formklingen;
e) Binden mindestens eines Abschnitts des einzelnen Blatts (114) an das Flüssigkeitsspeicherelement (14), um mindestens einen Abschnitt des einzelnen Blatts (114) als mindestens ein Flüssigkeitstransportelement (12) zu formen, das in der Lage ist, sich weg von dem Flüssigkeitsspeicherelement (14) radial zu erstrekken;
f) Falten des mindestens einen Flüssigkeitstransportelements (12) um eine zu der Längsachse (x-x) des Flüssigkeitsspeicherelements (14) parallele Achse; und

g) Verpacken der resultierenden intravaginalen Vorrichtung (10).

10. Apparat zur Herstellung einer intravaginalen Vorrichtung (10), der Folgendes umfasst:

    a) ein Formwerkzeug (102), das Folgendes umfasst:

        (i) eine Halteplatte mit einer Vielzahl von Vakuummündungen (116), die auf einer Fläche (118) davon geformt sind;
        (ii) eine im Wesentlichen kreisförmige Öffnung, die auf der Platte angeordnet ist, mit einer Vielzahl von Schlitzen (110), die mit ihr verbunden sind und sich von ihr erstrekken;

    b) ein Positivwerkzeug (104), das eine Vielzahl von Formklingen (106) umfasst, wobei jede Klinge eine Führkante aufweist, die um eine Öffnung (112) radial angeordnet sind;
    c) mindestens ein Bindeelement (108), das zu der Öffnung (112) in dem Formwerkzeug (102) hin bewegbar ist; und
    d) mindestens eine Stößelstange, die durch die Öffnungen (112) von jedem aus dem Formwerkzeug (102) und dem Positivwerkzeug (104) bewegbar ist;

    wobei die Öffnungen (112) des Formwerkzeugs (102) und des Positivwerkzeugs (104) entlang einer Maschinenachse ausgerichtet sind, um der Stößelstange zu erlauben, das Flüssigkeitsspeicherelement (14) durch jede Öffnung (112) zu bewegen, wobei die Formklingen (106) des Positivwerkzeugs (104) an den Schlitzen (110) des Formwerkzeugs (102) ausgerichtet sind und die Führkanten der Formklingen (106) angeordnet und eingerichtet sind, ein Flüssigkeitsspeicherelement (14) aufzunehmen, das an der Öffnung (112) des Positivwerkzeugs (104) ausgerichtet ist.

11. Apparat nach Anspruch 10, wobei jede Formklinge (106) eine Faltkante (112') aufweist, die ein Blatt (114) greifen kann, das auf der Fläche (118) der Halteplatte gehalten ist.

12. Apparat nach Anspruch 11, wobei die Faltkante (112') zu der Führkante benachbart ist.

13. Apparat nach Anspruch 11, wobei die Faltkante (112') von der Führkante durch mindestens einen Zwischenabschnitt der Formklingen (106) getrennt ist.

14. Apparat nach Anspruch 10, wobei das mindestens eine Bindeelement (108) in einer zu der Fläche der Halteplatte (118) parallelen Ebene bewegbar ist.

15. Apparat nach Anspruch 14, wobei das mindestens eine Bindeelement (108) bewegbar ist, um sich in einen Raum zwischen benachbarten Formklingen (106) zu erstrecken, wenn das Formwerkzeug (102) und das Positivwerkzeug (104) in einer Eingriffstellung stehen.

16. Apparat nach Anspruch 10, wobei das mindestens eine Bindeelement (108) ein Thermobonding-Element ist.

17. Apparat nach Anspruch 10, wobei das Flüssigkeitsspeicherelement (14) eine Länge aufweist und die Führkante von jeder Formklinge (106) eine Länge aufweist, die nicht größer ist als die Länge des Flüssigkeitsspeicherelements (14).

18. Apparat nach Anspruch 10, wobei das Flüssigkeitsspeicherelement (14) eine Länge aufweist und die Führkante von jeder Formklinge (106) eine Länge aufweist, die größer ist als die Länge des Flüssigkeitsspeicherelements (14).

**Revendications**

1. Dispositif de gestion de fluide (10) destiné à être utilisé dans un organisme mammifère, le dispositif comprenant :

    a) un élément de stockage de fluide (14) ayant un axe longitudinal (x - x), une extrémité d'insertion (48) et une extrémité de retrait (50) ; et
    b) au moins un élément de transport de fluide (12) qui est en communication de fluide avec l'élément de stockage de fluide (14), le au moins un élément de transport de fluide (12) comprenant :

        (i) une première plaque (18) ayant une surface orientée vers l'extérieur (34) et une surface orientée vers l'intérieur (30) ;
        (ii) une deuxième plaque (20) couplée à la première plaque (18) qui a une première surface (32) disposée et maintenue en relation de vis-à-vis avec la surface orientée vers l'intérieur (30) de la première plaque (18) et une surface opposée (36) ; et qui est capable de se séparer suffisamment de la première plaque (18) pour fournir l'action capillaire entre les plaques (28) ;

    dans lequel le au moins un élément de transport de fluide peut se courber autour d'un axe sensiblement parallèle à l'axe longitudinal (x - x) de l'élément de stockage de fluide.

**2.** Dispositif selon la revendication 1, dans lequel l'élément de transport de fluide (12) comprend un film perforé.

**3.** Dispositif selon la revendication 1 ou la revendication 2, dans lequel l'élément de transport de fluide (12) est fixé (54) sur l'extrémité de retrait (50) de l'élément de stockage de fluide (14).

**4.** Dispositif selon la revendication 3, dans lequel l'élément de transport de fluide (12) est fixé sur la base de l'élément de stockage de fluide (14).

**5.** Dispositif selon la revendication 1 ou la revendication 2, dans lequel l'élément de stockage de fluide (14) a au moins un côté longitudinal entre les extrémités d'insertion (48) et de retrait (50) ; et l'élément de transport de fluide (12) est fixé sur le au moins un côté longitudinal de l'élément de stockage de fluide (14).

**6.** Dispositif selon la revendication 1 ou la revendication 2, dans lequel l'élément de stockage de fluide (14) a au moins un côté longitudinal entre les extrémités d'insertion (48) et de retrait (50) ; et l'élément de transport de fluide (12) est fixé sur lui-même à proximité de l'extrémité de retrait (50) de l'élément de stockage de fluide (14).

**7.** Dispositif selon la revendication 1 ou la revendication 2, dans lequel l'élément de stockage de fluide (14) a au moins un côté longitudinal entre les extrémités d'insertion (48) et de retrait (50) ; et dans lequel l'élément de transport de fluide est fixé sur une ficelle de retrait (16) pour envelopper sensiblement l'élément de stockage de fluide (14).

**8.** Dispositif selon la revendication 5, 6 ou 7, dans lequel l'élément de transport de fluide (12) est fixé sur l'extrémité de retrait (50) de l'élément de stockage de fluide (14).

**9.** Procédé pour produire un dispositif intravaginal (10) comprenant les étapes consistant à :

a) séparer une feuille individuelle (114) d'une alimentation de matériau ayant des propriétés utiles pour déplacer les fluides corporels ;
b) prévoir un élément de stockage de fluide (14) ayant un axe longitudinal (x - x), des côtés longitudinaux, une extrémité d'insertion (48) et une extrémité de retrait (50) ;
c) mettre en prise la feuille individuelle (114) avec l'extrémité d'insertion (48) de l'élément de stockage de fluide (14) et avec des bords plissés (44) des lames de formage ;
d) pousser la feuille individuelle (114) à travers un outil de formage (102) avec le mouvement relatif de l'outil de formage (102) par rapport à l'élément de stockage de fluide (14) et les bords plissés (44) des lames de formage ;
e) relier au moins une partie de la feuille individuelle (114) à l'élément de stockage de fluide (14) afin de former au moins une partie de la feuille individuelle (114) en au moins un élément de transport de fluide (12) capable de s'étendre radialement à distance de l'élément de stockage de fluide (14) ;
f) plier le au moins un élément de transport de fluide (12) autour d'un axe parallèle à l'axe longitudinal (x - x) de l'élément de stockage de fluide (14) ; et
g) emballer le dispositif intravaginal (10) résultant

**10.** Appareil pour produire un dispositif intravaginal (10) comprenant :

a) un outil de formage (102) comprenant :

(i) une plaque de support ayant une pluralité d'orifices de vide (116) formés dans l'une de ses faces (118) ;
(ii) une ouverture sensiblement circulaire disposée sur la plaque ayant une pluralité de fentes (110) raccordées à et s'étendant à partir de cette dernière ;

b) un outil mâle (104) comprenant une pluralité de lames de formage (106), chaque lame ayant un bord de guidage, agencé radialement autour d'une ouverture (112) ;
c) au moins un élément de liaison (108) mobile vers l'ouverture (112) dans l'outil de formage (102) ; et
d) au moins une tige de poussée mobile à travers les ouvertures (112) de chacun parmi l'outil de formage (102) et l'outil mâle (104) ;

dans lequel les ouvertures (112) de l'outil de formage (102) et de l'outil mâle (104) sont alignées le long d'un axe de machine pour permettre à la tige de poussée de déplacer l'élément de stockage de fluide (14) à travers chaque ouverture (112), les lames de formage (106) de l'outil mâle (104) sont alignées avec les fentes (110) de l'outil de formage (102) et les bords de guidage des lames de formage (106) sont agencés et configurés pour loger un élément de stockage de fluide (14) aligné avec l'ouverture (112) de l'outil mâle (104).

**11.** Appareil selon la revendication 10, dans lequel chaque lame de formage (106) a un bord plissé (112') pouvant se mettre en prise avec une feuille (114) maintenue sur la face (118) de la plaque de support.

12. Appareil selon la revendication 11, dans lequel le bord plissé (112') est adjacent au bord de guidage.

13. Appareil selon la revendication 11, dans lequel le bord plissé (112') est séparé du bord de guidage par au moins une partie intermédiaire de la lame de formage (106).

14. Appareil selon la revendication 10, dans lequel le au moins un élément de liaison (108) est mobile dans un plan parallèle à la face de la plaque de support (118).

15. Appareil selon la revendication 14, dans lequel le au moins un élément de liaison (108) est mobile pour s'étendre dans un espace entre les lames de formage (106) adjacentes lorsque l'outil de formage (102) et les outils mâles (104) sont dans une position mise en prise.

16. Appareil selon la revendication 10, dans lequel le au moins un élément de liaison (108) est un élément de liaison thermique.

17. Appareil selon la revendication 10, dans lequel l'élément de stockage de fluide (14) a une longueur, et le bord de guidage de chaque lame de formage (106) a une longueur qui n'est pas supérieure à la longueur de l'élément de stockage de fluide (14).

18. Appareil selon la revendication 10, dans lequel l'élément de stockage de fluide (14) a une longueur et le bord de guidage de chaque lame de formage (106) a une longueur qui est supérieure à la longueur de l'élément de stockage de fluide (14).

Fig. 1a

Fig. 3a

Fig. 3b

Fig. 3c

## Fig. 1b

## Fig. 1c

32

42

18

20

40

40

30

# Fig. 2

# Fig 5

14

18

18

20

46

20

# Fig. 4a

Fig. 4b

Fig. 4d

Fig. 4e

# Fig. 6a

# Fig. 6b

Fig. 7

Fig. 8

Fig. 9

## Fig. 11

## Fig. 12

60

14

44

12

46

64

18

20

62

66

66'

64

60

60

58

# Fig. 14

# Fig. 13

Fig. 15

Fig. 16

Fig. 18

Fig. 19

**Fig. 17**

112

106

122

122

106

120

104

**Fig. 20**

68

10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4212301 A, Johnson
- US 6358235 B, Osborn
- US 6177608 B, Weinstrauch
- US 6206867 B, Osborn
- US 5817077 A, Foley
- US 5545155 A, Hseih
- US 1926900 A, Haas
- US 3811445 A, Dostal
- US 3422496 A, Wolff
- US 6310296 B, Friese
- US 5911712 A, Leutwyler
- US 3983875 A, Truman
- US 6554814 B, Agyapong
- US 3845766 A, Zoller
- US 5295984 A, Contente
- US 3929135 A, Thompson
- US 5567376 A, Turi
- US 4381326 A, Kelly
- US 20020123731 A1, Yang
- US 6570055 B
- US 5165152 A
- US 4816100 A, Friese
- US 6310269 B, Friese